# EUROPEAN PATENT APPLICATION

(11) **EP 2 060 284 A1**
(43) Date of publication of application: **20.05.2009**
(21) Application number: 07021984.5
(22) Date of filing: 13.11.2007
(51) Int. Cl.: A61M 5/14, A61M 5/24, A61M 5/31

(54) **Medical injection device having data input means and a pivotable display**

(71) Applicant: F.HOFFMANN-LA ROCHE AG, 4070 Basel (CH); Roche Diagnostics GmbH, 68305 Mannheim (DE)
(72) Inventor: Blasberg, Peter, 69469 Weinheim (DE); Koehler, Matthias, 69514 Laubenbache (DE); Rasch-Menges, Juergen, 69514 Schwetzingen (DE); Remde, Axel, 3432 Lützelflüh-Goldbach (CH); Heiniger, Hanspeter, 4932 Lotzwil (CH); Mouline, Blasise, 30700 La Capelle-Masmolene (FR)
(74) Representative: Poredda, Andreas

(57) **Abstract**

Device for the self-administration of liquid drugs in adjustable doses, the device being designed to be held in a hand for drug administration, the device comprising: a) a dosing module and an electronics module, the electronics module comprising: data entry means (10), the data entry means (10) being adapted for the entry of therapy related data, a memory (20), the memory (20) being adapted for storing the dose amount of administered doses and for storing therapy related data, and a data interface (40), the data interface (40) being adapted to download data stored in the memory (20) to an external device.

## Description

The following invention is related to devices for the self-administration of liquid drugs in adjustable doses

In the therapy of insulin-dependent diabetes mellitus, pen-shaped administration devices for the self-administration of insulin in adjustable doses are widely used and well known in the art. Such devices may be designed for ejecting a single, pre-installed insulin reservoir and to be disposed after fully emptying the reservoir. Such a device is exemplarily disclosed in WO 97/36625. Other devices are designed for multiple use whit the insulin reservoir being exchanged after emptying. Such devices may be of a more complicated design and may be equipped with an electronics module, e.g., for dose displaying purposes, such as the device disclosed in WO 93/16740.

In US 5536249, a pen-shaped administration device is disclosed, the adminsitration device comprising an injection pen adopted for the self-administration of insulin in adjustable doses, the dose amounts being stored in an administration device memory along with additional information, especially a timestamp indicating the time of administration. The administration device may further comprise a data interface for downloading data stored in the administration device memory to an external device for record keeping purposes and may further comprise an integrated blood characteristics monitor, such as a blood glucose monitor, wherein measured blood characteristics may be stored in the administration device memory together with a timestamp and may be downloaded via the administration device data interface to an external device.

In a state-of-the-art diabetes therapy, such as the 'intensified therapy', further factors such as the times and amounts of meal intakes and sportive activities, are variable in a wide range and have to be considered besides blood glucose levels and insulin administrations for performing the therapy, therapy recording and therapy evaluation. It is a well recognized problem that complete and reliable therapy records are often missing because the procedure of record-keeping is cumbersome and often not followed by a diabetic person. This results in incomplete, wrong and sometimes completely misleading or missing therapy records.

It is a first objective of the present invention to provide devices for the self-administration of liquid drugs which are compact, simple to handle and allow reliable and complete therapy recording.

A device according to the invention is designed to be held in a hand for drug administration and comprises a dosing module, the dosing module comprising a manually driven dosing mechanism, a drug reservoir, a housing enclosing the dosing mechanism and the drug reservoir, the housing defining a device axis L, and a dose setting element for setting a dose amount of a drug dose to be administered. In preferred embodiments, the housing is pen-shaped and the device axis L is the longitudinal pen axis. The device further comprises an electronics module, the electronics module comprising data entry means, the data entry means being adapted for the entry of therapy related data. The electronics module further comprises a memory, the memory being adapted for storing the dose amounts of administered doses and for storing therapy related data. The electronics module further comprises a data interface, the data interface being adapted to download data stored in the memory to an external device.

In preferred embodiments, the liquid drug is insulin and the data entry means is adapted for the entry of carbohydrate amounts and may be adapted for the entry of further therapy related data such as a level and duration of sportive activities. The drug reservoir is preferably a standard insulin cartridge with a volume of, e.g., 1.5ml or 3ml. In other embodiments, however, a device according to the present invention may be used for the administration of other drugs, such as pain relievers, and the therapy related data may comprise other kinds of information, such as pain levels.

For determining a dose amount which is set via the dose setting element, the device preferably comprises an encoder unit, the encoder unit being operatively coupled to the dosing mechanism and being operatively coupled the electronics module, the encoder unit generating an electrical signal reflecting the dose amount which is set via the dose setting element. In especially preferred embodiments, the encoder unit comprises an encoder transmitter and a encoder receiver. The encoder receiver can be realized by a set of electrical contacts, while the encoder transmitter can comprise a corresponding set linear came followers driven by a crank shaft, a set of came discs, or the like and be adapted to selectively operate the electrical contacts. Similarly, optical encoders or magnetic encoders with magnets and hall sensors may be employed. The encoder unit may, e.g., be designed in accordance with the disclosure of WO 93/16743.

In preferred embodiments, the data entry means are separate form the dose setting element. While the dose setting element of the dosing module is preferably realized as a rotary dose setting knob, the data entry means of the electronics module are preferably formed by a set of micro pushbuttons, which may be partly arranged as a crossbar switch, but may also comprise a joystick, a rotary data entry wheel, multi-position rotational switches or the like. In further embodiments, at least one ring may be arranged around the device housing, the at least one ring being manually rotatable around the device housing in at least one rotational direction, wherein rotating the at least one ring may be used for data entry. A number of several manually rotatable rings may especially be provided, wherein at least one of the rings may be rotated from a neutral rotational position in either of a first rotational operation position and a second rotational operation position.

The data which may be entered via the data entry means preferably comprise at least one quantitative value. In especially preferred embodiments, the data entry means are adapted for both increasing and decreasing a numeric value while being entered via the data entry means. If a device according to the present invention is used in the framework of diabetes therapy, the therapy related data preferably comprise food intake data, especially carbohydrate amounts, but may also comprise other data such as a type classification of meals, sportive activities, illnesses, data concerning the intake of additional drugs, or the like. More generally, all kind of data which are related to appropriate drug administration and are desirably stored for record-keeping and therapy evaluation purposes are therapy related data and may be entered via the data entry means.

The memory may be of several kinds known in the art, such as an electrically erasable read only memory or a dynamic random access memory but are preferably a static random access memory.

In preferred embodiments, the electronics module comprises a controller which is realized as Application Specific Integrated Circuit (ASIC) in especially preferred embodiments but may also have the form of a standard micro controller, discrete electronics, or the like. Further components of the electronics module may be integral with the controller.

In preferred embodiments, the electronics module comprises a clock circuit and the memory stores data together with a time stamp, the time stamp being generated by the clock circuit.

In some preferred embodiments comprising a clock circuit, the time stamps generated by the clock circuit comprise a date portion and a time-of-day portion, with the time of day being preferably stored with a resolution of one minute or one second. In especially preferred embodiments, however, the time stamp is indicates the difference between the current time and a reference time, the reference time being stored in an external device. For this kind of embodiments, the clock circuit may comprise a time counter rather than a real time clock, the time counter continuously counting with a time resolution of, e.g., I sec and the time stamps being given by the counter readings. The counter reading may be read out to an external device and subsequently be reset via the data interface. This procedure may be performed automatically, e.g., whenever communication between the administration device and an external device is established an/or along with downloading data from the memory to an external device. The external devices preferably comprises a real time clock, the real time clock counting the absolute time and storing the absolute time as reference time along with resetting the time counter. Based on the time resolution of the time counter, the time counter reading and the reference time stored along with the previous time counter reset, the absolute time corresponding to any time stamp stored in the memory can be computed. In this context, the term 'absolute time' refers to time with respect to a fixed point of time in history, such as midnight of Jan. 1st, 1900.

In preferred embodiments, the electronics module comprises a power supply which may comprise a single use battery, a rechargeable battery, a capacitor, a solar cell, or the like, or any combination of those. Depending on power consumption, power supply capacity and power supply lifetime in relation to electronics module lifetime, the power supply may or may not be replacable and may or may not be rechargable.

Some kinds of data interfaces, such as USB interfaces, provide a power supply. In especially preferred embodiments comprising a data interface comprising a power supply, the preferably present power supply can be charged via the data interface.

In preferred embodiments, the electronics module comprises information output means. In especially preferred embodiments, the information output means are adapted to output dose amounts and to output data which are entered via the data entry means and/or data which are derived from those data. If, for example, a carbohydrate amount is being entered in grams of carbohydrates, the output means may output the carbohydrate amount and/or the corresponding number of bread exchange units and/or or a dose amount of an insulin dose which is required to cover the entered carbohydrate amount.

In especially preferred embodiments, the information output means are capable of outputting numeric values and may especially comprise a display, e.g. a numeric or alphanumeric display, such as a Liquid Crystal Display (LCD). However, it may also be adapted to output semi-quantitative data such as a grade of sportive activity or a grade of illness, names of additional drugs, or the like. In especially preferred embodiments, the display is further capable of displaying several selectable menu items and/or functions which may be selected via the data entry means.

Additionally or alternatively to a display, other types of information output means may be provided, such as acoustic indication means, e.g. buzzers, or tactile indication means, such as pager vibrators. In these cases, numeric values such as carbohydrate amounts may be outputted as a sequence of indications, e.g. beeps and/or tactile indications, with each single indication representing a given increment of the numeric value to be outputted.

In especially preferred embodiments, the device comprises an administration volume counter, the administration volume counter being initialized when a fresh drug cartridge is taken into usage and calculating a cumulative drug dose amount as sum of the dose amounts of all doses ejected from the drug reservoir. The remaining drug volume is calculated as the difference of initial drug volume in the cartridge and cumulative drug dose amount. The remaining drug volume and/or the drug volume already ejected from the drug reservoir may be displayed on the preferably present display permanently and/or before and/or after administration a dose and/or on demand. A warning may further be displayed automatically if the remaining drug volumes falls below a remaining volume threshold. The remaining volume threshold may, e.g., correspond to a remaining volume of 20IU (international units) and may be factory set or configured by the user and/or a healthcare professional via the data entry means and/or via the data interface. The administration volume counter may be initialized manually by the user/and/or may be initialized automatically. In preferred embodiments the dosing mechanism is designed e.g., according to the disclosure of WO 93/16740 and comprises a pushing rod, the pushing rod taking a defined retracted position for a full reservoir. For this kind of embodiment, a full cartridge may be detected, e.g., by an end switch, the end being actuated when the pushing rod is in its fully retracted position.

In preferred embodiments, the electronics module comprises an orientation detector, the orientation detector signal indicating the orientation of the electronics module with respect to the gravity vector g., wherein the operation of the electronics module is partly controlled by the orientation detector signal.

A device according to the present invention may take several situation-specific orientations with respect to gravity. For drug administration, the device is typically oriented with the tip of the cannula pointing downwards. However, drug may also be ejected from the drug reservoir with the tip of the cannula pointing upwards for pressing air bubbles out of the drug reservoir and/or for priming the cannula prior to an injection. In this latter case, the corresponding drug dose is a priming dose and is therapeutically not relevant and should therefore not be stored in the memory as therapeutically relevant dose. Furthermore, for dose setting via the dose setting element as well as for entering data via the data entry means, the device is typically held in a horizontal position with the tip of the cannula pointing to the left or the right, depending on the user's individual habits. If the information shown on a display having a longitudinal axis parallel to the longitudinal device axis L is correct for the tip of the cannula pointing leftwards, it would appear upside-down and mirror-inverted for the tip of the cannula pointing rightwards, and vice versa. If the data input means, defines, at least in part, distinct operational directions, such as a crossbar switch, a joystick or arrow pushbuttons, the assignment of operational directions to functions is changed with respect to gravity, too. If, e.g., the arrow shown on a pushbutton points upwards for the cannula pointing rightwards, the arrow points downwards for the cannula pointing leftwards.In preferred embodiments, the orientation detector signals controls all or a number of orientation dependent functions, such as the orientation of data shown on a preferably present display, the assignment of operational defined by the data entry means to functions and the distinction between a therapeutically relevant drug dose and a therapeutically not relevant priming dose.

In especially preferred embodiments, a therapeutically not relevant priming dose amount may either be stored in the memory together with a time stamp and a flag, the or may not be stored in the memory.

In alternative embodiments, a dose can be manually indicated as therapeutically not relevant priming dose. For this purpose, a priming function may be activated via the data entry means or a dedicated priming button is provided.

In especially preferred embodiments, the orientation detector comprise two binary tilt switches. The binary tilt switches are preferably arranged with their longitudinal axes in a common plane, the common plane being parallel to the device axis L. The longitudinal axes of the tilt switches preferably include an angel of approximately 120° with the device axis L as bisector of the angle included by the longitudinal axes of the tilt switches.

Dependent on the specific situation and/or the user's individual habits, however, the output of the orientation detector may not reflect the actual situation. For example, a therapeutically relevant drug dose may be administered into the buttocks with the cannula pointing upwards, which would result in the dose being assumed to be therapeutically not relevant. Therefore, the user may override the orientation detector signal and/or the orientation detector may be completely be deactivated by the user in especially preferred embodiments.

In some preferred embodiments, the data interface comprises a wireless interface such as a BLUETOOTH Radio Frequency (RF) interface and/or a serial infrared interface, for example according to the IRDA standard. In further preferred embodiments, the data interface comprises a wired interface such as a Universal Serial Bus (USB) interface and/or a standard serial interface according RS232.

In preferred embodiments, the administration device comprises an identification code, the identification being stored in the electronics module, the identification code uniquely identifying the electronics module. The identification code preferably comprises a serial number of the electronics module. The identification code is preferably, at least in part, factory set and can not be modified after setting.

In especially preferred embodiments, the identification code further comprises drug type data. The drug type data may comprise, e.g., the exact drug name and/or information concerning the action characteristics of the drug.

In especially preferred embodiments, the data entry means are adapted for the entry of at least part of the drug type data. Alternatively or additionally, the entry of drug type data via the data entry means, drug type data may be uploaded from an external device via the data interface. In alternative embodiments, the drug type information is red in from the drug reservoir. In this type of embodiment, the drug reservoir may store drug type data in an RF-ID tag attached to the reservoir, as code comprising of a series of conducting and non-conducting areas which are red-in by corresponding contacts of the electronics module, as number of pins and/or grooves, the pins and/or grooves opening/or closing corresponding detection switches of the electronics module, or the like. If the drug is insulin, the drug type data preferably comprise information characterizing the insulin a fast-acting, regular or long-acting insulin.

In addition or alternatively to the data download via the data interface, especially preferred embodiments allow to review the information stored in the electronics module memory via the data output means preferably contained by the electronics module.

Besides downloading data stored in the memory, the data interface is preferably adapted to upload data from an external device, such as a PC or a PDA, to the electronics module in especially preferred embodiments. Those data may, for example, contain setup data such as required for time and date setting of the preferably present clock circuit and/or drug type data.

The data interface may especially be used to upload therapy related data from an external device to the electronics module. In the framework of diabetes therapy, for example, a data base run on a PDA, a cell phone or another portable device may be used to determine the carbohydrate amount of a selected type of meal. This carbohydrate amount may subsequently be uploaded to the electronics module via the data interface rather than manually entering the carbohydrate amount via the data entry means of the electronics module. In preferred embodiments, dose amount data can be uploaded to the electronics module via the preferably present data interface. The dose amount data, such as a dose amount to cover a specified meal, can be calculated by an external device for this type of embodiment.

In preferred embodiments, the electronics module comprises a dose calculator for calculating a dose amount from therapy related data. In the framework of diabetes therapy, the dose calculator may calculate insulin dose amounts based on a first set of patient-specific and/or time-of-day dependent factors, the factors specifying the dose amount to cover a given carbohydrate amount. The first set of factors is preferably uploaded to the electronics module as configuration data form an external device via the preferably present data interface but may also be entered via the data entry means of the electronics module. A dose amount calculated by the dose calculator may subsequently be set via the dose setting element and administered.

Besides covering carbohydrate intake, diabetics occasionally have to administer insulin to correct undesirably increased blood glucose values. Accordingly, glucose values as measured by a commercially available blood glucose meter may preferably be entered into the electronics module via the data entry means and a second set of patient-dependent and/or time-of-day dependent factors specifying the dose amount required to correct a given raise in blood glucose may be used by the dose calculator in order to calculate an appropriate dose amount of a correction insulin dose. In other embodiments, the device comprises determining means such as a blood glucose meter, the determining means being part of the electronics module or operatively coupled to the electronics module.

In especially preferred embodiment, an external device capable of uploading therapy related data to the electronics module consists of a blood glucose meter, contains a blood glucose meter or may be operationally coupled to a blood glucose meter. In this case, measured blood glucose values may be directly uploaded from the external device to electronics module rather than manually entering blood glucose data via the data entry means. Similarly, a dose amount required to correct a given increase in blood glucose may be calculated by the external device and uploaded to the electronics module via the preferably present data interface.

The administration of drugs such as short acting insulin or regular insulin is dependent on therapy related data. However an administration device according to the present invention allows for dose setting and administration without prior entry of corresponding therapy related data via the data entry means and/or transferring corresponding therapy related data from an external device to the administration device. In preferred embodiments, a warning is given to the user via the preferably present data output means and/or additional indication means if no corresponding therapy related data are being entered along with drug administration.

Furthermore, data such as carbohydrate amounts may be entered without a corresponding drug administration. While this may happen on intention in some cases, it is also known to happen mistakenly. Accordingly, a warning is preferably given to the user via the preferably present data output means and/or additional indication means if a drug dose is not being administered along with entering therapy related data normally accompanied by a drug administration.

In some preferred embodiments, the electronics module is fully integral with the dosing module and all components of the electronics module are comprised by the device housing. In other preferred embodiments, the electronics module comprises a display, the display being comprised by a display housing. The display housing is pivoting about a pivoting axis S. In especially preferred embodiments, the pivoting axis S is substantially transversal to the device axis L. A display comprised by a pivoting display housing is advantageous with respect to ergonomic one-handed operation. For this type of embodiment, the display can be pivoted to an optimal reading position virtually independent from the device orientation.

In especially preferred embodiments, the display housing is adapted to be pivoted into a storing orientation, such that the data entry means are concealed if the display housing is in the storing orientation. The data entry means may especially be arranged such that the display housing covers the data entry means if the display housing is in the storing orientation.

In some preferred embodiments, the electronics module and the dosing module are detachably coupable via coupling means. This configuration allows to use one and the same electronics module in combination with a number of dosing modules or vice verse, for example if the lifetime of one of the modules is shorter than the lifetime of the other module. For this kind of embodiment, the preferably present encoder unit is partly comprised by the dosing module and partly comprised by the electronics module in especially preferred embodiments. The encoder unit may, e.g., comprise a set of came followers, the came followers serving as encoder transmitter, the came followers projecting from a contact surface of the electronics module and selectively operating corresponding electrical contacts, the electrical contacts serving as encoder receiver, the encoder receiver being comprised by the electronics module. For this kind of embodiment, the elements of the encoder unit which are comprised by the dosing module are referred to as encoder transmitter while the elements of the encoder unit which are comprised by the electronics module are referred to as encoder receiver independent of the specific encoder embodiment.

In especially preferred embodiments, the electronics module and the dosing module are locked with respect to each other in the attached state. In especially preferred embodiments, a detachably coupable electronics module comprises a pivoting display as described above. wherein the coupling may be unlocked by pivoting the display housing. Furthermore, at least one defined detaching orientation of the display housing is provided and detaching the electronics module is only possible with the display housing in the at least one detaching orientation in especially preferred embodiments.

In some especially preferred embodiments, the drug reservoir is permanently enclosed by the device housing during the manufacturing process and cannot be subsequently removed without damaging the device hosing. Accordingly, the dosing module is disposed after emptying the drug reservoir while the electronics module is reused. Designing the dosing module to be a single use device and to be disposed after emptying the drug reservoir is especially desirable from a user comfort point of view because it eliminates the need for drug reservoir exchanges which is often found to be cumbersome and difficult

In especially preferred embodiments, drug type data are stored in the dosing module in an RF-ID tag, wherein the information stored in the RF-ID tag are red-in by the electronics module, in code comprising of a series of conducting and non-conducting areas which are red-in by corresponding contacts of the electronics module, in number of pins and/or grooves, the pins and/or grooves opening/or closing corresponding detection switches of the electronics module, or the like. In further preferred, different types of dosing modules and electronics modules are provided, wherein the releasable coupling means are indicative for a given drug type and are adapted to enable only coupling of corresponding dosing modules and electronics modules, wherein the drug reservoir of the corresponding dosing module stores the drug corresponding to the electronics coupling means. The Drug type data are preferably readily stored in the electronics module for this type of embodiment.

Because a dosing module is generally not designed to be used without electronics module, devices comprising releasbly coupable dosing modules and electronics modules preferably comprise locking means, such as a mechanical locking mechanism, to lock the dose setting element if the electronics module is not coupled to the dosing module.

For devices comprising releasbly coupable dosing modules and electronics modules, with the data interface being a wired data interface such as a USB connector or RS232 connector, the coupling means may, at least in part, be defined by the connector.

It is a second objective of the present invention to provide administration devices for the self-administration of liquid drugs in adjustable doses which are designed for ergonomic one-handed operation.

An administration device for ergonomic one-handed operation according to the present invention comprises a dosing module, the dosing module comprising a manually driven dosing mechanism, a drug reservoir, a device housing enclosing the dosing mechanism and the drug reservoir and a dose setting element, the device housing defining a device axis L. The administration device further comprises an electronics module, the electronics module comprising data entry means and information output means. The information output means comprise a display, such as a LCD, and may further comprise additional information output means, such as a buzzer and/or a pager vibrator. The administration device further comprises a display housing and an electronics module housing. The display housing is pivoting about a pivoting axis S, the pivoting axis S being different from a device axis L. In especially preferred embodiments, the pivoting axis S is transversal to the device axis L.

Preferred embodiments of an administration devices according to the second objective of the present invention are describes above with respect to an administration device according to the first aspect of the invention and may belong to an administration device according to the second aspect of the invention as well.

It is a third objective of the present invention to provide electronics modules, the electronics modules being detachably coupable to an administration unit to form a hand-held administration device.

An electronics module according to the present invention comprises data entry means. The data entry means are adapted for manual data entry and are adapted for the entry of therapy related data in especially preferred embodiments. The electronics module further comprises electronics module coupling means, the electronics module coupling means being adapted for detachably coupling the electronics module to a dosing module, the dosing module comprising a drug reservoir, a manually driven dosing mechanism and a dose setting element. The Electronics module further comprises a encoder receiver, the encoder receiver receiving a signal from n encoder transmitter, the encoder transmitter being comprised by the dosing module, the signal reflecting the dose amount of a drug dose which is set via the dose setting element (5) of the dosing module (4). The electronics module further comprises a memory, the memory being adapted for storing the dose amount of administered doses and for storing therapy related data and a data interface, the data interface being adapted to download stored in the memory to an external device.

In combination, the electronics module according to the present invention and the dosing module form an administration device, the administration device being adapted to be held in a hand for drug administration.

In preferred embodiments, the electronics module coupling means are indicative for a given drug type and the dosing module coupling means are adapted to enable coupling only with a corresponding dosing module, the drug reservoir of the corresponding dosing module storing the drug corresponding to the electronics module coupling means.

In the framework of diabetes therapy, the different drugs may, e.g. different types of insulin which may be supplied by different manufacturers and/or may have different action characteristics. Providing coupling means which are indicative for the drug type allows the user to use several combinations of electronics modules and dosing modules in parallel, wherein the drug reservoirs of the different dosing modules may comprise different drugs and to ensure appropriate coupling of corresponding electronics modules and dosing modules.

In preferred embodiments, drug type data are stored in the dosing module in an RF-ID tag, wherein the information stored in the RF-ID tag are red-in by the electronics module, in a code comprising of a series of conducting and non-conducting areas which are red-in by corresponding contacts of the electronics module, in number of pins and/or grooves, the pins and/or grooves opening/or closing corresponding detection switches of the electronics module, or the like. This kind of embodiment allows the electronics module to securely identify the drug type without the deed to provide multiple types of coupling means.

In preferred embodiments, the electronics module comprises a display and a display housing comprising the display and the display housing is pivoting about a pivoting axis S with respect to an electronics housing, wherein the coupling can be unlocked by pivoting the display housing. In especially preferred embodiments, at least one defined unlocking orientation of the display housing is provided. Providing a pivoting display is preferable under ergonomic and device size aspects and unlocking the coupling by pivoting the display housing both a simple and robust design of the coupling means while avoiding the need for further user operated control elements.

Further aspects of preferred embodiments of an electronics module according to the third objective of the present invention are describes above with respect to an administration device according to the first and the second aspect of the invention and may belong to an electronics module according to the third aspect of the invention as well.

In the following, several exemplary embodiments for administration devices according to the present invention are described in greater detail and with reference to the Figures. While all exemplary embodiments are exemplary embodiments for an administration device according to the first aspect of the invention, both the second exemplary embodiment and the third exemplary embodiment are exemplary embodiments for an administration device according to the second aspect of the present invention. Furthermore, the electronics module of the third exemplary embodiment is an exemplary embodiment of an electronics module according to the third aspect of the present invention.
Figure 1 shows a first example of device for the self-administration of liquid drugs in accordance with the present invention.
Figure 2 shows a structural diagram of the electronics module of the device shown in Figure 1.
Figure 3a to 3d show orientation detection means in different orientations with respect to gravity.
Figure 4 shows a first configuration of a second example of a device for the self-administration of liquid drugs in accordance with the present invention.
Figure 5 shows a second configuration of a second example of a device for the self-administration of liquid drugs in accordance with the present invention.
Figure 6 shows a first configuration of a third example of a device for the self-administration of liquid drugs in accordance with the present invention.
Figure 7 shows a second configuration of a third example of a device for the self-administration of liquid drugs in accordance with the invention.

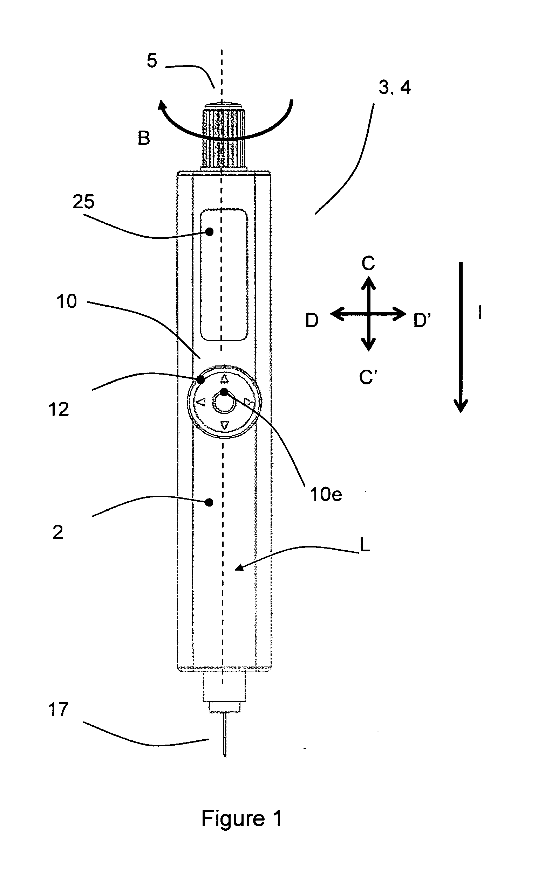

Figure 1 shows a first example of a device for the self administration of liquid drugs in accordance with the invention. It is especially suited for the administration of insulin, but may used for the administration of other liquid drugs, too.

Most components of the device are enclosed by a pen-shaped device housing 2 having a device axis L. An injection cannula 17 projects from device housing 2 into the direction indicated by vector 1, vector 1 being parallel to the device axis L. The cannula 17 is coupled to the device housing 2 via releasable cannula coupling means (not shown).

The cannula 17 is typically disposable and should be used for a single injection for sterility reasons. In other embodiments, the cannula 17 may be used for several injections. A cover (not shown in Fig. 1) may be provided to cover the cannula coupling means or a cannula 17, respectively.

A Dose setting knob 5 serves as dose setting element and may project from device housing 2. The dose setting knob 5 is normally in a retracted position inside the device housing 2 and may be extended by pressing a release key (on the backside of device housing 2, not shown in Figure 1). In its extended position, the dose setting knob 5 may be rotated clockwise into rotational direction B for increasing a dose amount to be administered or may be rotated anti-clockwise (against rotational direction B) for decreasing a dose amount to be administered. While rotating the dose setting knob 5, the dose amount is displayed on display 25 in real time. The minimum dose amount increment is defined by rotational catching positions corresponding to, e.g., 0.5 IU (International Units), while a full rotation of dose setting knob 5 corresponds to, e.g. a dose amount of 20 IU. After setting a dose to be administered, the patient may grip the device with one hand and perform the injection by (i) piercing the skin of, e.g., an upper arm or thigh by moving the device into direction 1 parallel to device axis L towards the skin, and (ii) driving a dosing mechanism to administer the insulin dose by slowly pressing the dose setting knob 5 down and back into its retracted position. The manually driven dosing mechanism may be designed according to the disclosure of WO 93/16740 or another suited design known in the art.

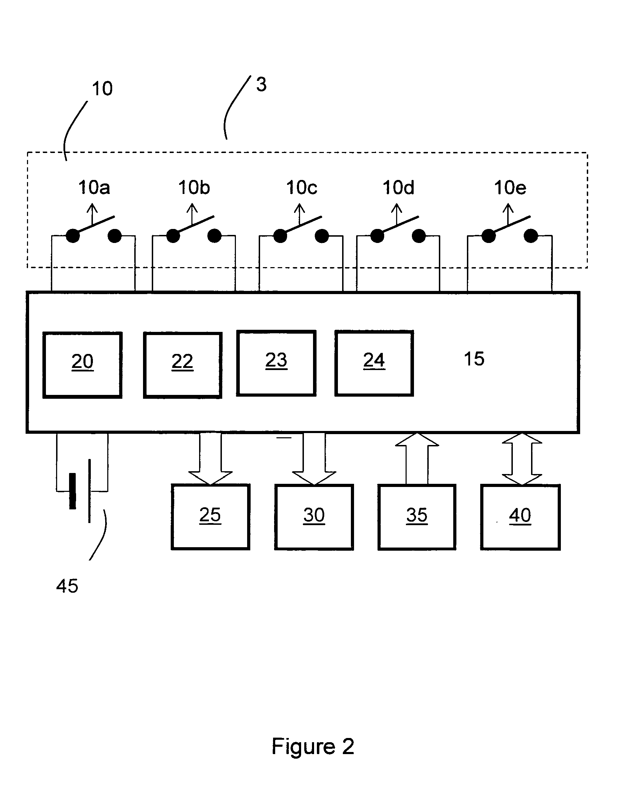

In the following, reference is made to Figure 1 as well as to Figure 2, Figure 2 showing a block diagram of the electronics module 3, the electronics module 3 being enclosed by the device housing 2 and fulfilling multiple functions. The core element of the electronics module 3 is a controller 15 which is realized as ASIC.

For scanning the rotational movement of the dose setting knob 5, an encoder unit is provided which detects the rotation of the dose setting knob 5 and transfers an corresponding electrical signal to the controller 15 dependent on the direction of rotation. The encoder unit comprises a set of came followers, the came followers serving as encoder transmitter. The came followers selectively operate interact with a corresponding set of electrical contacts 35, the contacts serving as encoder receiver 35. The encoder unit may, e.g., be of the design disclosed in WO 93/16743. A preferred encoder embodiment is described in some detail below in the context of another exemplary embodiment of an administration device.

The controller 15 is operatively coupled with data output means (25, 30). The Liquid crystal display (LCD) 25 is adapted for showing numeric data and application specific data. When rotating the dose setting knob 5, the accumulated dose amount is computed by the controller 15 based on the signals generated by the encoder receiver 35 and is displayed on the display 25. During drug administration, the dose amount displayed on display 25 is counted down, indicating the dose amount still to be administered.

Besides display 25, an acoustic buzzer 30 is provided as additional data output means especially for notification and indication purposes.

When actually administering the insulin, the accumulated dose amount is stored in memory 20 together with a time stamp, the time stamp being generated by clock circuit 22, the time stamp comprising a time of day portion and a date portion. Both the memory 20 and the clock circuit 22 are integral with controller 15.

Data entry means 10 are provided especially for entering carbohydrate amounts and other types of numerical information. In this exemplary embodiment, the data entry means 10 are realized by a number of 5 micro pushbuttons 10a, 10b, 10c, 10d, 10e. The pushbuttons 10a, 10b are used for function selection, while the pushbuttons 10c, 10d, are used for increasing or decreasing a numeric value to be entered and may be also used for selecting items from a menu , or the like. The pushbutton 10e operates as ENTER key for completing data input. Besides carbohydrate amounts, additional therapy related data such as sportive activities may be entered via the data entry means 10 in a similar way. The type of data to be entered can be selected using pushbuttons 10a, 10b.

The electronics module 3 further comprises a power supply in form of a replacable or non-replacable battery 45. The electronics module 3 changes from a standby state to a regular operation state whenever the dose setting knob 5 is extended or any of the pushbuttons 10a, 10b, 10c, 10d, 10e is pressed.

Changes from the regular operation state into the standby state occur if no operation is being performed for a threshold time of, e.g. 2 minutes.

After changing from the standby state to the regular operation state, the display 25 shows the dose amount of the latest administered dose in the past and the remaining drug volume in the drug reservoir. These data may be display simultaneously, sequentially or alternating for a given period of time or until the next operation is performed, i.e., dose setting knob 5 is rotated or a either of the pushbuttons is pressed. Displaying the dose amount of the latest dose administered in the past is helpful to prevent unintended double delivery.

For calculating the remaining drug volume in the drug reservoir, the electronics module 3 comprises an administration drug volume counter (not shown in the figures), the administration drug volume counter being initialized via the data entry means 10 when a fresh drug cartridge is taken into usage and calculating a cumulative drug dose amount as the sum of all drug amounts ejected from the drug reservoir. The remaining drug volume is calculated as the difference of initial drug volume in the cartridge and the cumulative drug dose amount.

The four pushbuttons 10a, 10b, 10c, 10d are arranged as a crossbar switch 12, with each of the four operation directions C, C', D, D' corresponding to one of the push buttons 10a, 10b, 10c, 10d. ENTER key 10e is arranged in the center of the crossbar switch 12. This arrangement allows for an easy one-handed operation.

Carbohydrate amounts are preferably entered in carbohydrate exchange units which are used by many diabetics. However, other units such as grams of carbohydrates may be used, too. The device may be configured by the patient or the health care professional for carbohydrate amount entry in the desired units.

In order to easily enter both large and small carbohydrate amounts with sufficient resolution, the function pushbuttons 10a, 10b allow to choose between a large carbohydrate increment I2 and a small carbohydrate increment I1. Besides the entry of both large and small carbohydrate amounts, this functions enables the patient to coarsely enter A carbohydrate amount using increment I2 and subsequent fine adjustment using increment I1. Generally, each single press on one of the push buttons 10c/10d increases/decreases the carbohydrate amount by an increment I1 or I2, respectively. However, a scroll function may also be implemented which allows to increase/decrease the carbohydrate amount as long as the corresponding pushbutton 10c/10d is being pressed continuously. If carbohydrate amounts are entered in carbohydrate exchange units, I1 may, e.g. correspond to 0.1 carbohydrate exchange unit and 12 may, e.g., correspond to 1.0 carbohydrate exchange unit.

After entering a carbohydrate amount, the entry is confirmed and completed by pressing the ENTER key 10e which results in the carbohydrate amount being stored in the memory 20 together with a time stamp, the time stamp being generated by the clock circuit 22. If the patient does not modify the entered carbohydrate amount nor presses the ENTER button 10e within a certain time after of, e.g. 5 sec, the entry is canceled.

The administration of insulin and the entry of carbohydrate amounts are generally independent form each other and may accordingly be stored in the memory 20 independently together with corresponding time stamps. So it is possible to only enter a carbohydrate amount without administering insulin. This situation may occur, e.g. when eating small snacks or when eating additional food during sportive activities. On the other hand, it is also possible to set and administer an insulin dose without food intake. This situation may occur when insulin is administered in order to compensate for undesirably increased blood glucose values.

In most cases, however, insulin administration and carbohydrate intake happen in combination. Here it is favorable to store both the insulin dose amount and the carbohydrate amount together with a common time stamp. For this purpose, a time window of e.g., 2 minutes is foreseen. If both a dose is administered and a carbohydrate amount is entered within this time window, both the dose amount and the amount of carbohydrates are stored in memory 20 with one common time stamp.

Besides carbohydrate data, other therapy related data, such as time, duration and intensity or sportive activities, illnesses or intake of additional drugs may be entered via the data entry means 10 to be stored in the memory 20. For entering those therapy related data, corresponding input modes may be selected via pushbuttons 10a, 10b.

In order to download the data stored in memory 20 to an external data processing unit, a data interface 40 is provided. The data interface 40 is realized as wireless infrared interface and/or BLUETOOTH RF interface. For downloading purposes, a DOWNLOAD HISITORY function is provided. The external data processing unit may be any device such as a personal computer (PC), a personal digital assistant (PDA) or a cell phone. It may also be a specialized diabetes management device including a blood glucose meter. The external data processing unit may be used for multiple purposes, such as long-term storage, printing logbooks and therapy data visualization. When downloading data from memory 20 to an external device, an identification code is downloaded along with the data. The identification code comprises the serial number of the electronics module 3 in order to enable unique identification.

The data interface 40 is not only used for history download, but may also be used to upload data from an external device to the administration device. It can especially be used to set configuration data such as the time and date in clock circuit 22 and/or the unit for entering carbohydrate amounts. Furthermore, the data interface 40 can be used to upload drug type data to the device. The drug type data are stored in electronics module together with the serial number of the electronics module 3 and are part of the identification code. Alternatively to uploading the drug type data via data interface 40, the drug type data may be entered via the data entry means 10.

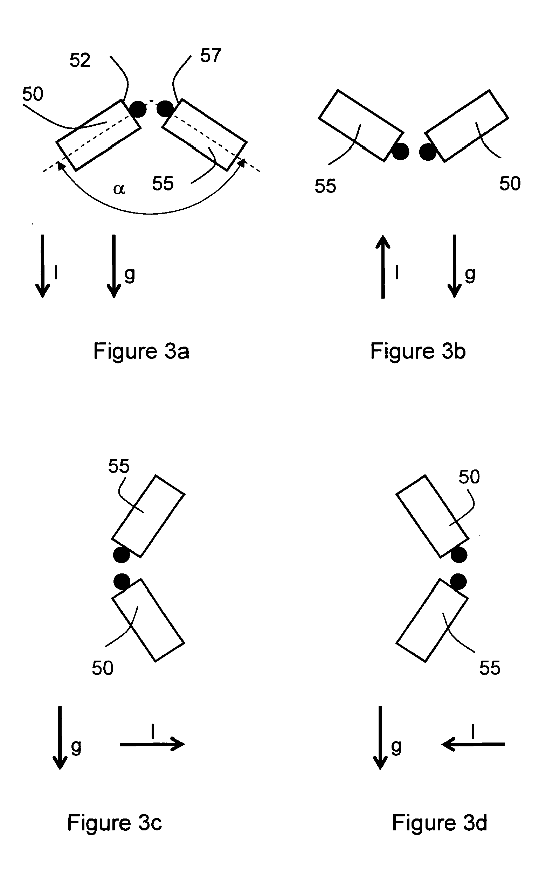

Some functions of the device depend on its orientation with respect to gravity. For determining the orientation of the device axis L with respect to gravity, two tilt switches 50, 55 of generally cylindrical shape are provided as shown in Figure 3a to Figure 3d. The tilt switches 50, 55 form an orientation detector and are coupled to the controller 15. The tilt switches 50, 55 are arranged inside the device housing 2 with their center axes being in a common plane generally parallel to the device axis L and including an angel *a* of about 120° with device axis L as bisector of the angle α .Each of the tilt switches 50, 55 comprises a contact which is closed if gravity vector g has a substantial component pointing towards reference front surface 52 of tilt sensing switch 50 or reference surface 57 of tilt switch 57, respectively and which is open otherwise. Four orientations of the administration device with respect to the gravity vector g may be distinguished based on the combined output signals of both tilt switches 50, 55 as reflected in Figure 3a to 3d.
a) Vector 1 parallel to gravity vector g (Figure 3a): Cannula 17 points downwards resulting in tilt switch 50 and tilt switch 55 being open.
b) Vector 1 anti parallel to gravity vector g (Figure 3b): Cannula 17 points upwards resulting in tilt switch 50 and tilt switch 55 being closed.
c) Vector 1 pointing 90° rightwards with respect to gravity vector g (Figure 3c): Cannula 17 points rightwards resulting in tilt switch 50 being open and tilt switch 55 being open.
d) Vector 1 pointing 90° leftwards with respect to gravity vector g (Figure 3d): Cannula 17 points backwards resulting in tilt switch 50 being closed and tilt switch 55 being open.

Distinguishing between these four cases a) to d) , the administration device is controlled as follows. Case a) is the typical situation when gripping the administration device with one hand and administering insulin for example into an upper arm or thigh. If insulin is being administered in this configuration, it is considered as injection and the dose amount is stored in the memory 20 together with a time stamp.

Case b) is the typical situation when air has to be forced out of the insulin reservoir and/or the cannula 17 has to be primed with insulin prior to an injection. These operations are performed by administering a small dose of insulin with the cannula 17 pointing upwards without penetrating the tissue. If a dose is being administered in this configuration, it is not considered as injection and a priming flag is stored in memory 20 along with the dose amount. However, the user may manually override the signal generated by the orientation detector (50, 55), i.e. the user may manually set the priming flag in cases the priming flag would not be set automatically or may cancel an automatically set priming flag. The override function is used to cope with situations in which the signal generated by the orientation detector (50, 55) would be misleading. This may happen dependent on the specific situation and/or the user's individual habits. Furthermore, the orientation detector (50, 55) may be disabled by configuration.

For entering a carbohydrate amount or performing other operations requiring operation of the data entry means 10, the administration device is typically held with the device axis L being horizontal while the cannula 17 may point rightwards (Figure 3c) or leftwards (Figure 3d), depending on the patient's individual habits. However, assuming that the information on display 25 is being displayed correctly for configuration c), it would be displayed upside down and mirror-inverted in configuration d) and vice versa. On crossbar switch 12, the arrow pointing upwards changes to pointing downwards and vice versa, while the arrow pointing left changes to pointing downwards and vice versa. Based on the information provided by the tilt switches 50 and 55, the controller 15 compensates this undesired effect by turning the display content by 180° as well as pairwise interchanging the function of pushbutton 10a with the function of pushbutton 10b and interchanging the function of pushbutton 10c with the function of pushbutton 10d.

Alternatively, the assignment of functions to the pushbuttons 10a, 10b, 10c, 10d and the orientation of the information on the display 25 may be configured manually.

Because insulin doses required to compensate for food intake are directly correlated with the carbohydrate amount, the required dose amount can be automatically calculated based on the carbohydrate amount entered via the data entry means 10. For this purpose, a dose calculator is provided by an algorithm on controller 15. Insulin dose amounts are calculated and displayed based on a set of patient-specific and time-of-day dependent factors. The set of factors is preferably uploaded to the electronics module from an external device via the data interface 40 but may also be entered via the data entry means 10.

In addition to the administration related functionality described above, the administration device comprises a countdown timer and an alarm clock functionality for reminder purposes. The alarm clock functionality may especially be used as reminder to perform a blood glucose test at a given time of day. The countdown timer may be triggered manually or may be triggered automatically when administering an insulin dose as reminder to perform a blood glucose test a given time of e.g., 1.5 hours after insulin administration. Access to the countdown timer and the alarm clock functionality is provided via the data entry means 10. The Alarm clock and countdown timer may give indications via the display 25 and/or the buzzer 30.

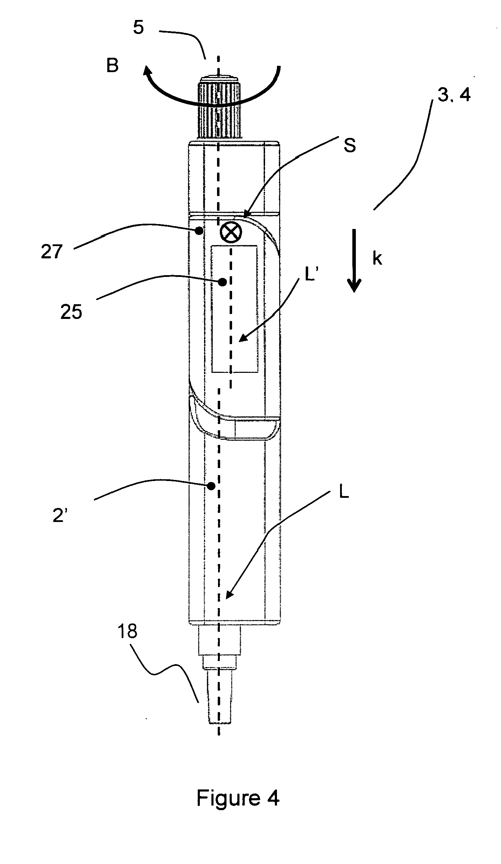

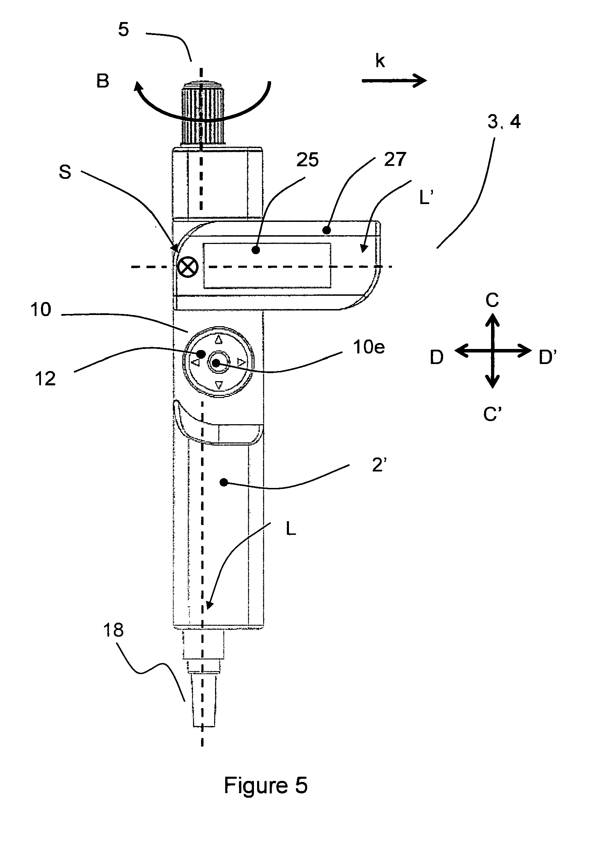

Figure 4 and Figure 5 show a second example of a device for the self administration of insulin in accordance with the invention. While many structural elements and most of the functionality are identical to the previous example, the design shown in Figure 4 and Figure 5 is especially suited for one-handed operation. In both Figure 4 and Figure 5, the cannula is enclosed by a cannula safety shield 18 and therefore not visible.

In this embodiment, the display 25 is comprised by a display housing 27 while the other components of the electronics module 3 are comprised by the device housing 2'. The display housing 27 has a display housing axis L' and is pivoting with respect to the device housing 2' about a pivoting axis S, with the pivoting axis S being transversal to the device axis L.

Both storing and transporting the device and administering insulin are performed with the display housing 27 being pivoted into a storing orientation as shown in Figure 4. In the storing orientation, a display housing vector k is aligned with the device axis L and points towards the cannula. The display housing vector k points from the pivoting axis S along the display 27 and is aligned with the display housing axis L'. In this configuration, the data entry means 10 are concealed by the display housing 27 for preventing damage as well as unintended operation.

For data entry via the data entry means 10, the display housing 27 can be pivoted into a user selected orientation, e.g. an orientation with the display housing axis L' being substantially transversal to the device axis L by a pivoting motion about the pivoting axis S as shown in Figure 5. In this configuration, one-handed operation is possible with one hand gripping device housing 2' and the thumb of the same hand operating the crossbar switch 12 as well as ENTER key 10e.

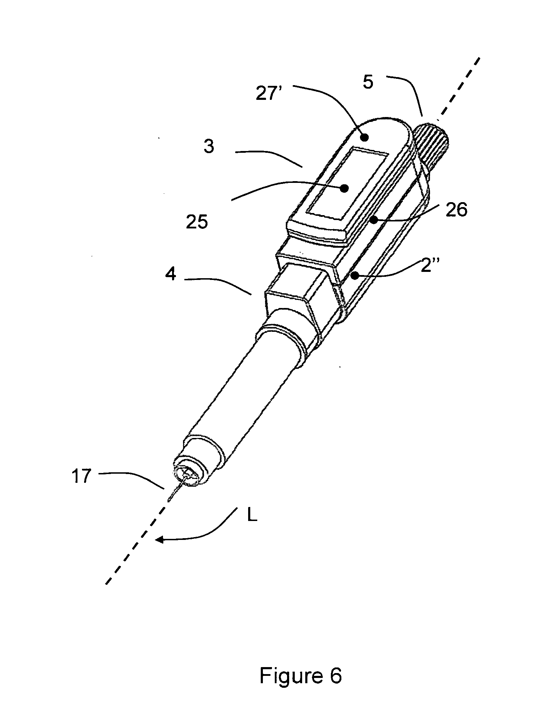

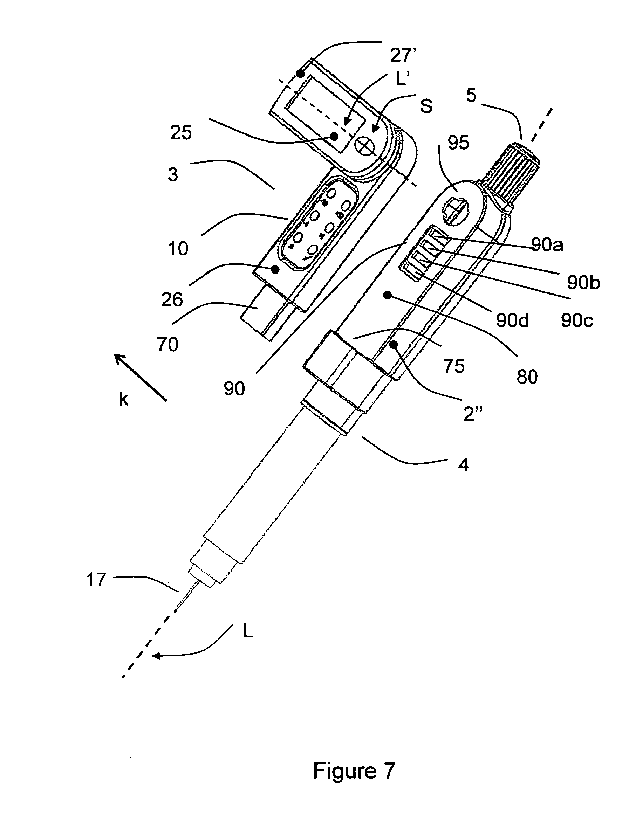

Figure 6 and Figure 7 show a third example of a device for the self administration of insulin in accordance with the invention. This embodiment is different from the embodiments described above insofar as it is designed to be modular with the electronics module 3 being detachably coupable with the dosing module 4. While the dosing module 4 designed to be disposed after emptying the insulin reservoir, electronics module 3 is adapted to be reusable. The device housing 2" of the dosing module 4 is supplied readily equipped with the insulin reservoir and the drive mechanism as one-way device. The insulin reservoir is permanently enclosed by the device housing 2" during the manufacturing process and cannot be subsequently removed without damaging the device hosing.

By this means, the handling comfort is considerably improved especially for visually or otherwise handicapped patients for whom changing reservoirs is cumbersome and difficult.

Figure 6 shows the device in the operational state with the electronics module 3 being coupled with the dosing module 4, while Figure 7 shows the decoupled state.

For coupling purposes, a housing contact face 80 is provided at the device housing 2" and a corresponding electronics module contact face (not visible) is provided on the back side of the electronics module housing 26.

The data interface 40 of the electronics module 3 is realized as a USB, having a standardized USB plug 70. For data transfer purposes, the USB plug 70 of detached electronics module 3 may be plugged into a corresponding USB socket of an external device such as a PC. An USB interface is especially suited for data download and data upload with virtually any commercial PC or similar device.

The energy supply 45 comprises a rechargeable battery and/or a capacitor. The rechargeable battery and/or the capacitor 45 may be recharged via the USB plug 70 with the electronics module 3 being detached from the dosing module 4.

For coupling the electronics module 3 and dosing module 4, detatachble coupling means 70, 75, 80, 95 are provided. For coupling, the electronics module 3 is pushed along housing contact face 80 with a plug 70 being inserted into a corresponding pocket 75 of the device housing 2'', the pocket 75 being a dummy standard USB socket. The coupling means further comprise a dosing module coupler 95 at face 80 and a corresponding electronics module coupler. The dosing module coupler 95 comprises an alignment member projecting from the dosing module contact face 80 while the electronics module coupler comprises a corresponding cutout in the electronics module contact face (not visible). The electronics module coupler further comprises a plug (not visible), the plug projecting from the electronics module contact face (not visible). The plug is pivoting together with the display housing 27' about the pivoting axis S. The Dosing module coupler 95 comprises a socket in face 80, the socket corresponding to the plug of the electronics module coupler. Both the shape of the plug, the plug being part of the electronics module coupler, and the shape of the socket, the socket being part of dosing module coupler 95, are designed such that the electronics module 3 and the dosing module 4 are not locked with respect to each other to allow detaching of the electronics module 3 if the display housing 27' is in a detaching orientation. In the detaching orientation, the display housing vector k is aligned with the device axis L and points towards the dose setting knob 5. In the detached state, a locking mechanism prevents the administration of insulin, the locking mechanism being integral with the dosing mechanism.

For transferring dose amount information from the dosing module 4' to the electronics module (3), an encoder transmitter 90 is provided, the encoder transmitter 90 comprising four came followers 90a, 90b, 90c, 90d which may project from the dosing module contact face 80 and close corresponding electrical contacts. The electrical contacts are arranged on the electronics module contact face (not visible in Figure 7) when projecting form the dosing module contact face 80. In combination, the electrical contacts form the encoder receiver 35. The Came follower 90a projects from dosing module contact face 80 if the dose setting knob 5 projects from the device housing 2'' and is retracted when the dose setting knob 5 is in its fully retracted position. Closing and opening of the corresponding contact is used to change the electronics module 3 between regular operation state and standby state as described above.

The came followers 90b, 90c, 90d are adopted to transfer dose amount information. Rotating the dosing knob 5 results in each of the came followers 90b, 90c, 90d to project for three consecutive dose amount increments followed by three consecutive dose amount increments in the retracted position. The change from the projecting state to the retracted state and from the retracted state to the projecting state, however, is not synchronous for the came followers 90b, 90c, 90d. The came follower 90c changes from its retracted state to its projecting state and vice versa one dose amount increment after the came follower 90b while the came follower 90d changes from its retracted state to its projecting state and vice versa one dose amount increment after the came follower 90c. Resulting from this arrangement, one of the came followers 90b, 90c, 90d changes its state either from its retracted to its projecting or from its projecting position to its retracted, every time the dose setting knob 5 is rotated about one increment, while the other came followers keep their previous state. By evaluating the order in which the corresponding electrical contacts are being opened and closed by the came followers 90b, 90c, 90d, the rotational direction of the dose setting knob 5 is being determined. While two came followers would generally be sufficient for transferring the dose amount information, three came followers are preferred for safety reasons. Except from providing four came follower fingers rather than three, the mechanical structure may be identical to the one disclosed in WO 93/16743.

Unlike the examples described above, the data entry means 10 comprise a set of single push buttons rather than a coordinate switch. Six pushbuttons are provided for increasing and decreasing numeric data such as carbohydrate amounts with a large increment I2, increasing and decreasing numeric entry data with a small increment I1, and for accepting and canceling data entries.

## Claims

1. Device for the self-administration of liquid drugs in adjustable doses, the device being designed to be held in a hand for drug administration, the device comprising:
a) a dosing module (4), comprising:
- a manually driven dosing mechanism,
- a drug reservoir,
- a device housing (2, 2', 2"), the device housing (2, 2', 2") enclosing the dosing mechanism and the drug reservoir, the housing (2, 2', 2") defining a device axis (L), and
- a dose setting element (5) for setting a dose amount,
b) an electronics module (3), comprising:
- data entry means (10), the data entry means (10) being adapted for the entry of therapy related data,
- a memory (20), the memory (20) being adapted for storing the dose amount of administered doses and for storing therapy related data,
- a data interface (40), the data interface (40) being adapted to download data stored in the memory (20) to an external device.

2. Device according to Claim 2, **characterized in that** the electronics module (3) comprises a clock circuit (22) and **in that** the memory (20) stores data together with a time stamp, the time stamp being generated by the clock circuit (22).

3. Device according to Claim 2, **characterized in that** the time stamps generated by the clock circuit 22 indicates the difference between the current time and a reference time, the reference time being stored in an external device.

4. Device according to either of the previous claims, **characterized in that** the electronics module (3) comprises information output means (25).

5. Device according to either of the previous claims, **characterized in that** electronics module (3) comprises an orientation detector (50, 55), the orientation detector (50, 55) generating an orientation detector signal, the orientation detector signal indicating the orientation of electronics module (3) with respect to gravity vector (g), wherein the operation of the electronics module (3) is partly controlled by the orientation detector signal.

6. Device according to Claim 6, **characterized in that** the orientation detector (50, 55) comprises two binary tilt switches (50, 55).

7. Device according to either of the previous claims, **characterized in that** the electronics module (3) comprises a dose calculator for calculating a dose amount from therapy related data.

8. Device according to either of the previous claims, **characterized in that** the data, interface (40) is adapted to upload data to the electronics module (3).

9. Device according to Claim 8, **characterized in that** the data that can uploaded to electronics module (3) can comprise dose amount data.

10. Device according to either of the previous claims, **characterized in that** the electronics module (3) comprises a display (25) and **in that** the display (25) is comprised by a display housing (27, 27'), wherein the display housing (27, 27') is pivoting about a pivoting axis (S).

11. Device according to Claim 10, **characterized in that** the display housing (27, 27') is adapted to be pivoted into a storing orientation, such that the data entry means (10) are concealed if the display housing (27, 27') is in the storing orientation.

12. Device according to either of the previous claims, **characterized in that** the electronics module (3) and the dosing module (4) are detachably coupable via coupling means (70, 75, 80, 95).

13. Device according to Claim 13, **characterized in that** the drug reservoir is permanently enclosed by the device housing (2").

14. Device according to Claim 12 or Claim 13, **characterized in that** the device comprises locking means, the locking means locking the dose setting element (5) if the electronics module (3) is not coupled to the dosing module (4).

15. Device according to either of Claim 12 to Claim 14, **characterized in that** the data interface (40) comprise a connector (70) and that the coupling means (70, 75, 80, 95) are at least partly defined by the connector (70).

16. Device according to either of the previous claims, **characterized in that** the electronics module (3) comprises a power supply (45), wherein the power supply (45) can be charged via the data interface (40).

17. Device according to either of the previous claims, **characterized in that** the liquid drug is insulin and that the data entry means (10) is adapted for the entry of carbohydrate amounts.

18. Device for the self-administration of liquid drugs in adjustable doses, the device being designed to be held in a hand for drug administration, the device comprising:
a) a dosing module (4) comprising
- a manually driven dosing mechanism,
- a drug reservoir,
- a device housing (2', 2'') enclosing the dosing mechanism and the drug reservoir, the device housing defining a device axis L,
b) an electronics module (3) comprising
- data entry means (10),
- information output means (25), the information output means comprising a display (25),
d) - a display housing (27, 27'), the display housing (27, 27') comprising the display (25) and pivoting about an pivoting axis (S), the pivoting axis (S) being different from the device axis (L).

19. Electronics module (3), comprising:
a) data entry means (10),
b) electronics module coupling means (70), the electronics module coupling means (70) being adapted for detachably coupling the electronics module (3) to a dosing module (4), the dosing module (4) comprising a drug reservoir, a manually driven dosing mechanism and a dose setting element (5),
c) an encoder receiver (35), the encoder receiver receiving a signal from an encoder transmitter (90), the encoder transmitter (90) being comprised by the dosing module (4), the signal reflecting the dose amount of a drug dose which is set via the dose setting element (5) of the dosing module (4"),
d) a memory (20), the memory (20) being adapted for storing the dose amount of administered doses and for storing therapy related data, and
e) a data interface (40), the data interface (40) being adapted to download data stored in the memory to an external device,
wherein the electronics module (3) and the dosing module (4) form an administration device, the administration device being adapted to be held in a hand for drug administration.

20. Electronics module according to either of Claim 19, **characterized in that** the electronics module coupling means (70) are indicative for a given drug type, and **in that** the electronics module coupling means (70) are adapted to enable coupling only with a corresponding dosing module (4), the drug reservoir of the corresponding dosing module (4) storing the drug corresponding to the electronics module coupling means (70).

21. Electronics module according to either of Claim 19 or Claim 20, **characterized in that** drug type data are transferred from the dosing module (4) to the electronics module (3) if the electronics module (3) is coupled to the dosing module (4).

22. Electronics module according to either of Claim 19 to Claim 21, **characterized in that** the electronics module comprises a display (25) and a display housing (27') comprising the display (25), the display housing (27') pivoting about a pivoting axis (S'), wherein the coupling can be unlocked by pivoting the display housing (27').
